# FASCICULE DE BREVET EUROPEEN

(11) **EP 0 596 800 B1**
(45) Date de publication et mention de la délivrance du brevet: **12.03.1997**
(21) Numéro de dépôt: 93402700.4
(22) Date de dépôt: 03.11.1993
(51) Int. Cl.: C07C 5/333, C07C 11/02, C07C 7/10

(54) **Procédé et dispositif de déshydrogénation catalytique d'une charge paraffinique C2+ comprenant des moyens pour inhiber l'eau dans l'effluent**
Verfahren und Vorrichtung zur katalytischen Dehydrierung einer C2+ paraffinischen Charge, einschliesslich eines Mittels zur Hemmung von Wasser im Ausfluss
Method and apparatus for the catalytic dehydrogenation of a paraffinic C2+ charge, including means for removing water from the effluent

(30) Priorité: 06.11.1992 FR 9213516
(43) Date de publication de la demande: 11.05.1994
(73) Titulaire: INSTITUT FRANCAIS DU PETROLE, 92502 Rueil-Malmaison (FR)
(72) Inventeur: Minkkinen, Ari, F-78860 Saint Nom la Breteche (FR); Burzynski, Jean-Pierre, F-69110 Sainte-Foy-les-Lyon (FR); Larue, Joshep, F-78240 Chambourcy (FR)

(56) Documents cités:
- EP-A- 0 007 750
- US-A- 3 536 775
- US-A- 3 663 641

## Description

L'invention concerne un procédé de déshydrogénation catalytique d'une charge hydrocarbonée paraffinique C₂+ et un dispositif de mise en oeuvre du procédé. Elle a trait plus spécialement à l'inhibition de l'eau contenue dans l'effluent avant la réfrigération et la séparation de l'effluent.

Les brevets US-A-3 536 775 et US-A-3 663 641 décrivent un procédé pour enlever par contact avec de l'eau l'oxygène contenu dans une coupe butadiène obtenue par une déshydrogénation oxydante d'une coupe butène. Le brevet EP-A-7750 décrit un procédé pour enlever l'eau contenue dans des hydrocarbures légers non oléfiniques tel que le gaz naturel, les gaz liquéfiés, des essences ou du kérosène, par ajout de méthanol aqueux. On obtient dans ces conditions une phase liquide hydrocarbonée contenant le méthanol qui empêche la formation d'hydrates. De plus, le méthanol n'est pas récupéré de la phase liquide aqueuse.

Enfin, le brevet US-A- 3 663 641 illustre l'arrière plan technologique.

Il est connu qu'un nombre de procédés industriels mettant en oeuvre des réactions catalytiques à basse pression opèrent dans un environnement d'hydrogène dans lequel la pression partielle en hydrogène est assurée par un recyclage d'un gaz riche en hydrogène contenu dans un effluent réactionnel et qui a été séparé des hydrocarbures.

C'est le cas notamment d'un procédé de déshydrogénation catalytique des GPL contenant du propane, du butane et de l'isobutane pour produire des monooléfines qui servent d'intermédiaire pour les carburants à haut indice d'octane. Dans le cas de la déshydrogénation de l'isobutane, l'isobutène produit peut réagir avec du méthanol pour produire du méthyltertiobutyléther, un additif utilisable dans les essences.

L'art antérieur est illustré par les brevets US 4381 418 et US 4381 417. Dans de tels procédés la réaction est réalisée dans un réacteur catalytique continuellement régénéré opérant à très faible pression (légèrement supérieure à la pression atmosphérique ou sous vide) et à des températures de 500 à 600°C.

L'hydrogène recyclé et l'hydrogène produit assurent une pression partielle en hydrogène suffisante pour inhiber la formation de coke et de ce fait maintenir la stabilité du catalyseur. On atteint ainsi une conversion satisfaisante à une gamme de températures plus élevée, avoisinant par exemple 600°C. En règle générale, l'effluent à basse pression délivré par la zone réactionnelle de déshydrogénation est refroidi d'abord par échange de chaleur avec la charge gazeuse puis avec de l'eau, à une température appropriée avant que la pression de vapeur de l'effluent ne soit efficacement remontée dans un équipement de compression conventionnel, à une pression supérieure, ce qui permet la séparation de l'hydrogène et des composés hydrocarbonés de l'effluent. Ces échangeurs traditionnels et autres refroidisseurs à air augmentent la contre-pression du système réactionnel, ce qui affecte négativement le taux de conversion. Pour remédier à ces inconvénients il a été tenté d'utiliser des échangeurs de chaleur à faible perte de charge et à refroidissement direct avec des liquides de refroidissement (de trempe) circulants, mais sans grand succès.

La séparation de l'hydrogène des hydrocarbures de l'effluent doit se réaliser à une pression supérieure à celle régnant dans la zone réactionnelle. De plus, pour condenser les hydrocarbures dans le mélange gazeux constituant l'effluent et contenant l'hydrogène, il est nécessaire de refroidir à une température inférieure à celle que peuvent atteindre les échangeurs de chaleur à l'air ou à l'eau traditionnels.

Puisqu'une réfrigération en-dessous de 0°C est requise pour la séparation de l'hydrogène et des composés hydrocarbonés, l'eau présente dans l'effluent comprimé doit être éliminée et atteindre une concentration limite telle qu'il n'y ait pas congélation, de façon à prévenir l'obstruction de l'équipement de réfrigération. On utilise à cette fin des tamis moléculaires 3 Å pour déplacer l'eau de l'effluent avant l'étape de réfrigération (1Å = 10⁻¹⁰ m).

Or, les lits d'adsorption à tamis moléculaires imposent aussi des pertes de charge sur l'effluent de compression dues non seulement aux lits d'adsorption eux-mêmes mais aux systèmes de filtres en aval de ces lits qui captent les poussières de tamis moléculaires susceptibles d'obstruer le passage de l'effluent dans les équipements de réfrigération. Par ailleurs, les tamis moléculaires impliquent des périodes d'adsorption de l'eau suivies de périodes de régénération qui sont difficiles à contrôler.

Un objet de l'invention est de remédier aux inconvénients mentionnés ci-dessus.

L'invention concerne donc un procédé de déshydrogénation catalytique d'une charge hydrocarbonée paraffinique C₂+ comprenant une étape de déshydrogénation de ladite charge en phase gazeuse, éventuellement en présence d'hydrogène, délivrant un effluent gazeux de déshydrogénation comprenant de l'eau, de l'hydrogène, des hydrocarbures oléfiniques et des hydrocarbures paraffiniques non convertis, au moins une étape de refroidissement de l'effluent, une étape de compression de l'effluent refroidi à une pression appropriée, une étape d'inhibition de l'eau contenue dans l'effluent comprimé, une étape de réfrigération dans un échangeur de chaleur de l'effluent comprimé, une étape de séparation de l'effluent comprimé et réfrigéré dans une zone de séparation dans des conditions adéquates et une étape de récupération d'hydrogène et d'hydrocarbures en majeure partie oléfiniques.

Plus particulièrement, le procédé est caractérisé en ce qu'on sature en eau une partie au moins de l'effluent refroidi dans des conditions adéquates dans une zone de saturation par contact direct avec un mélange de phases liquides contenant en majeure partie de l'eau, avant ou après l'étape de compression et on effectue l'étape d'inhibition de l'eau dans l'effluent:
a) en contactant dans une zone de strippage et dans des conditions adéquates une partie au moins de l'effluent comprimé et saturé en eau avec une phase liquide recyclée renfermant à la fois de l'eau et un solvant dans des proportions appropriées, ledit solvant étant un composé organique nonhydrocarbure, normalement liquide, autre que l'eau, ledit composé étant au moins partiellement miscible à l'eau et distillable à une température inférieure à celle de la température d'ébullition de l'eau, de façon à obtenir une phase gazeuse chargée de solvant et d'effluent et une phase liquide aqueuse sensiblement débarrassée de solvant ;
b) en introduisant la phase gazeuse chargée de solvant et d'effluent dans la partie restante de l'effluent comprimé et saturé en eau, lorsque la totalité de l'effluent comprimé et saturé en eau n'a pas été mis en contact avec la phase liquide recyclée ;
c) en faisant circuler la phase gazeuse chargée de solvant et d'effluent de l'étape b) ou de l'étape a) lorsque l'étape b) n'est pas nécessaire, dans ledit échangeur de chaleur lors de l'étape de réfrigération ;
d) en séparant dans la zone de séparation une phase liquide décantée contenant de l'eau et du solvant avec une concentration appropriée et
e) en récupérant et en recyclant la phase liquide décantée de l'étape d) vers ladite colonne de strippage de l'étape a).

En réalisant la saturation en eau de l'effluent en amont du dispositif de compression, on effectue avantageusement une trempe directe de l'effluent et on évite de ce fait la présence de refroidisseurs à air avant la compression. Par ailleurs, la majeure partie des impuretés contenues dans l'effluent sont éliminées, ce qui évite de plus d'introduire une étape de lavage de l'alimentation en amont de l'unité de MTBE. Enfin, on minimise les pertes de charge en amont du compresseur.

En effectuant la saturation en eau de l'effluent en aval du dispositif de compression selon une variante du procédé, on réalise aussi une trempe directe de l'effluent et on élimine en outre la majeure partie des impuretés contenues dans l'effluent avec les avantages décrits ci-avant.

Selon une caractéristique du procédé, on peut effectuer efficacement la saturation de l'effluent refroidi en introduisant à partie supérieure de la zone de saturation la phase liquide aqueuse récupérée de la colonne de strippage et une phase aqueuse recyclée récupérée en fond de la zone de saturation, en introduisant à la partie inférieure de la zone de saturation l'effluent refroidi, en réalisant un contact direct à contre-courant de l'effluent avec le mélange des phases aqueuses à travers un garnissage approprié, en récupérant la phase aqueuse en fond de la zone de saturation que l'on recycle, après l'avoir refroidie au moins une fois, à la partie supérieure de ladite zone, et on récupère dans la partie supérieure de la zone de saturation l'effluent saturé en eau et refroidi.

En général, le mélange de phases aqueuses introduites dans la zone de saturation est à une température de -10 à +90°C. Il peut avantageusement renfermer de 1 à 15 % d'un hydroxyde de métal alcalin et de préférence de la soude.

La température de la colonne de saturation est généralement de 0 à 100°C et avantageusement de 20 à 50°C. On opère habituellement dans cette colonne sous une pression pouvant aller de la pression atmosphérique à 3 bar absolus et plus particulièrement de la pression atmosphérique à 1,5 bar absolus, lorsque la colonne pour saturer l'effluent en eau est disposée entre le réacteur de déshydrogénation et l'équipement de compression de l'effluent. Dans le cas où elle est située après la compression de l'effluent, la colonne de saturation peut opérer sous une pression de 3 à 35 bar absolus et de préférence 8 à 15 bar absolus. La pression dans la colonne de saturation peut donc varier de 1 à 35 bar absolus (1 bar = 10⁵Pa).

Le strippage du solvant dans la colonne de strippage est en règle générale effectué à une température de 10 à 150°C, de préférence 80 à 120°C sous une pression de 3 à 35 bar absolus et de préférence 10 à 15 bar absolus.

L'étape de réfrigération de l'effluent, réalisée par un dispositif d'autoréfrigération (vaporisation de la charge liquide avec ou sans hydrogène recyclé) ou par un dispositif de réfrigération externe, peut être effectuée à une température de 0 à -85°C, de préférence -20 à -40°C sous une pression de 3 à 35 bar absolus.

Enfin, la séparation de l'effluent en hydrogène, en oléfines et en la phase liquide eau et méthanol qui est décantée est réalisée généralement sous 3 à 35 bar absolus, de préférence sous 8 à 12 bar absolus et à une température de 0 à -100°C et plus particulièrement de -20 à -40°C.

Selon une caractéristique du procédé, pour réaliser efficacement le strippage du solvant dans la colonne de strippage, on peut introduire 1 à 100 % en poids de l'effluent comprimé comme fluide de strippage et de préférence 10 à 50 %.

Cette colonne de strippage comprend habituellement un garnissage de préférence structuré, de hauteur équivalente à au moins 1 plateau théorique, par exemple 1 à 20 et avantageusement 10 à 15 plateaux. Ces conditions combinées avec l'introduction d'une proportion variable d'effluent dans la colonne de strippage font que l'eau récupérée en fond de colonne de strippage et recyclée à l'entrée de la zone de saturation peut renfermer au plus 10 000 ppm de solvant, avantageusement moins de 2 000 ppm et de préférence 50 à 500 ppm.

Selon une autre caractéristique du procédé, la concentration de méthanol dans la phase décantée eau-méthanol provenant de l'étape de réfrigération de l'effluent qui est introduite dans la colonne de strippage est en règle générale de 20 à 80 % en poids, et de préférence 50-65 %. Généralement, on mesure la densité du solvant dans l'eau constituant la phase décantée, ce qui permet d'obtenir sa concentration et par comparaison avec une valeur de référence stockée dans un micro-ordinateur, on peut ajuster cette concentration au niveau souhaité par l'ajout d'un flux contenant du solvant dans l'effluent comprimé.

Ce flux peut être avantageusement le solvant ou les eaux de lavage (méthanol) d'une unité aval de production de méthyltertiobutyléther.

Le solvant utilisé doit être au moins partiellement miscible avec l'eau. De préférence, il doit avoir une température d'ébullition inférieure à celle de l'eau ou former avec l'eau un azéotrope dont la température d'ébullition est inférieure à celle de l'eau, de manière à pouvoir être entraîné par la phase gazeuse constituant l'effluent au cours de l'étape de mise en contact du procédé. Il doit aussi être peu soluble dans les hydrocarbures.

Ce solvant peut être avantageusement le méthanol et l'éthanol et de préférence le méthanol en raison de son faible coût. Il peut être également choisi par exemple parmi les solvants suivants : méthylpropyléther, éthylpropyléther, dipropyléther, méthyltertiobutyléther, diméthoxyméthane, diméthoxyéthane, éthanol, méthoxyéthanol, propanol.

L'invention concerne aussi l'unité de déshydrogénation catalytique comprenant en combinaison un réacteur (40) de déshydrogénation délivrant un effluent contenant de l'eau, des moyens d'alimentation (12, 2) en une charge hydrocarbonée connectés à une entrée du réacteur, au moins un moyen de refroidissement (41) d'un effluent relié à une sortie du réacteur, des moyens de compression (6) de l'effluent, des moyens d'inhibition de l'eau contenue dans l'effluent, des moyens de réfrigération (13) de l'effluent comprimé reliés aux moyens d'inhibition de l'eau, des moyens de séparation (8) de l'effluent réfrigéré reliés aux moyens de réfrigération, des moyens de récupération (22, 18) d'une phase riche en hydrogène et d'une phase liquide contenant des hydrocarbures oléfiniques,

Plus particulièrement l'unité est caractérisée en ce qu'elle comporte :
- des moyens de saturation en eau d'une partie au moins de l'effluent comprenant une enceinte (3) ayant un garnissage approprié, de forme allongée qui comporte à une extrémité une première entrée reliée à une alimentation (1) en effluent, une première sortie d'eau reliée à des moyens de recyclage (42, 45) de l'eau, à l'autre extrémité une deuxième entrée reliée aux moyens de recyclage (42) de l'eau et une deuxième sortie (5) délivrant l'effluent saturé en eau, et
- les moyens d'inhibition de l'eau comportant une colonne (10) de strippage ayant un garnissage adéquat, de préférence structuré, et ayant à une première extrémité une première entrée (11) de l'effluent saturé en eau reliée à la deuxième sortie de l'enceinte (3) de saturation en eau de l'effluent, une première sortie reliée aux moyens (42a) de recyclage de l'eau vers l'enceinte (3) de saturation et à une extrémité opposée une deuxième entrée reliée à des moyens de recyclage (9) d'une phase liquide aqueuse contenant du solvant connectés auxdits moyens de séparation (8) adaptés à délivrer ladite phase liquide aqueuse et une deuxième sortie (47) connectée aux moyens de réfrigération (13) de l'effluent.

Selon une première variante de l'unité, la première entrée de l'enceinte de saturation est reliée auxdits moyens (41) de refroidissement de l'effluent, la deuxième sortie de l'enceinte de saturation est reliée à une entrée des moyens de compression (6) et la première entrée de l'effluent saturé en eau dans la colonne (10) de strippage est connectée à une sortie (7) des moyens de compression.

Par ailleurs, la sortie des moyens de compression peut être reliée en outre aux moyens de réfrigération de l'effluent lorsque la totalité de l'effluent ne passe pas à travers la colonne de strippage.

Selon une deuxième variante de l'unité, les moyens de refroidissement de l'effluent en sortie du réacteur peuvent être connectés à une entrée des moyens de compression, la première entrée de l'enceinte (3) de saturation est reliée à une sortie des moyens de compression (6) et la deuxième sortie de l'enceinte de saturation (3) est reliée directement à la première entrée de la colonne (10) de strippage.

Par ailleurs, la deuxième sortie de l'enceinte de saturation peut être connectée en outre aux moyens de réfrigération lorsque la totalité de l'effluent ne passe pas à travers la colonne de strippage.

En règle générale, la charge peut comprendre des hydrocarbures paraffiniques à 3, 4 et/ou 5 atomes de carbone. Plus particulièrement, elle peut comprendre de l'isobutane.

Elle peut être introduite sous forme liquide ou sous forme gazeuse, en présence ou non d'hydrogène recyclé.

Par rapport aux unités de déshydrogénation selon l'art antérieur, la présente invention présente l'avantage d'exiger des niveaux de pression, aussi bien dans le réacteur de déshydrogénation que dans les dispositifs de compression, bien plus faibles en raison des faibles pertes de charge dues aux dispositifs de saturation en eau et donc de quench direct de l'effluent, et d'inhibition de l'eau par l'utilisation des solvants. Il en résulte dans ces conditions des taux de conversion supérieurs à ceux de l'art antérieur dans la mesure où l'on peut opérer dans le réacteur de déshydrogénation à des niveaux de pression plus faibles.

Les dispositifs de réfrigération de l'effluent en vue de sa séparation en gaz (hydrogène) et en liquide (oléfines et hydrocarbures paraffiniques non convertis) peuvent être externes (échange indirect avec le propane par exemple).

Selon une autre variante particulièrement avantageuse, ils peuvent comprendre un échangeur de chaleur à double enceinte, dont la première est alimentée par la charge liquide sans ou avec une partie au moins de l'hydrogène recyclé provenant des moyens de séparation, cet hydrogène pouvant par ailleurs être détendu avant son entrée dans la première enceinte de l'échangeur de chaleur. La vaporisation de la charge avec ou sans hydrogène contribue alors à la réfrigération de l'effluent dans la seconde enceinte de l'échangeur de chaleur (tubes ou plaques par exemple).

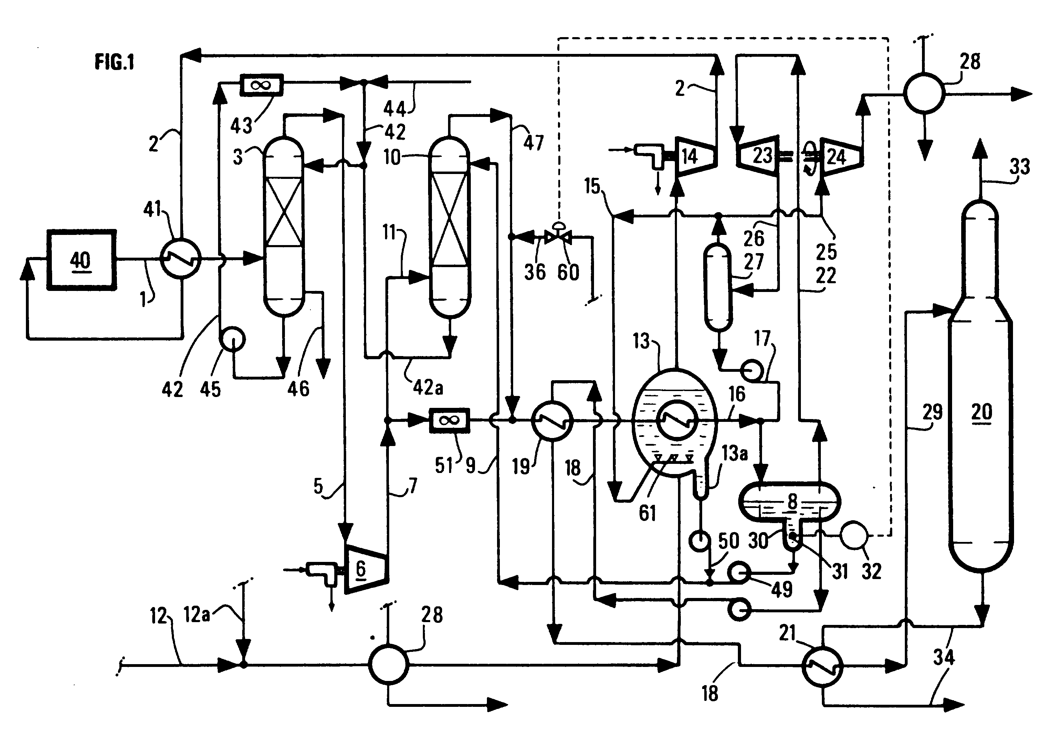

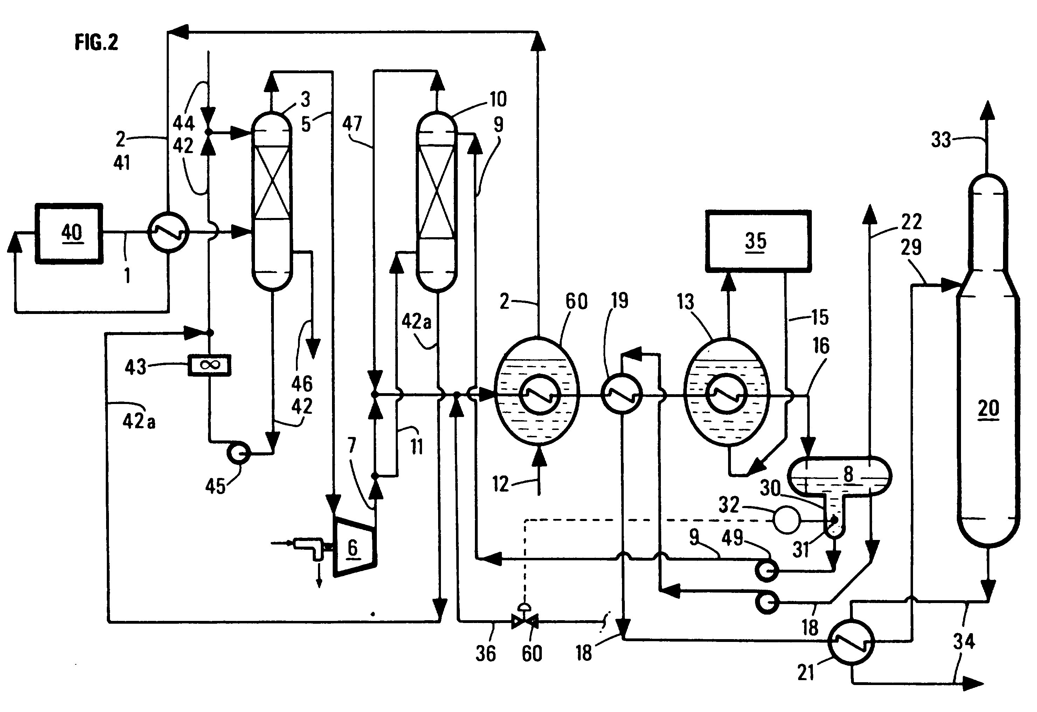

L'invention sera mieux comprise au vu des figures 1 et 2 illustrant de manière schématique le procédé et le dispositif selon l'invention parmi lesquelles :

La figure 1 représente une unité de déshydrogénation d'une coupe contenant de l'isobutane à 93 % comprenant le dispositif d'inhibition de l'eau selon l'invention et un système d'autoréfrigération de l'effluent.

La figure 2 montre une unité de déshydrogénation comprenant le dispositif d'inhibition de l'eau selon l'invention et un système de réfrigération externe de l'effluent.

La charge isobutane 2, sous forme gazeuse et de l'hydrogène, après avoir été préchauffés à une température appropriée par échange direct dans un échangeur de chaleur à plaques 41, à faible perte de charge, sont introduits dans un réacteur 40 de déshydrogénation catalytique fonctionnant à basse pression (1,5 à 1,8 bar absolus) et à haute température 580-600°C. L'effluent 1 qui en sort enrichi en hydrocarbures oléfiniques (plus de 50% par exemple) est refroidi par échange indirect dans cet échangeur à plaques, puis dans un autre échangeur 3 qui est une colonne à garnissage structuré à grande surface spécifique et de hauteur équivalente à environ 5 plateaux théoriques. Cette colonne réalise une trempe directe de l'effluent à une température d'environ 20 à 50°C, sature l'effluent en eau et le débarrasse de ses impuretés.

L'effluent est introduit dans la colonne par sa partie inférieure, traverse le garnissage de bas en haut où il est mis en contact à contre-courant avec un mélange de phases liquides contenant de l'eau introduite dans la partie supérieure de la colonne, provenant pour partie d'une ligne de recyclage 42 à la base de la colonne 3 via une pompe 45 et au moins un échangeur à air 43 et pour la partie restante d'une colonne de strippage 10 à partir d'une ligne d'alimentation 42a. De la soude (5 % en poids environ) peut être introduite par un conduit 44 dans la ligne 42 pour laver l'effluent de ses impuretés indésirables.

De l'eau peut être soutirée de la partie inférieure de la colonne de quench et de saturation par une ligne 46. L'effluent refroidi à environ 20-50°C mais encore surchauffé est récupéré en tête de colonne et alimente par une ligne 5 l'aspiration d'un système de compression 6 centrifuge, à une pression sensiblement supérieure à la pression atmosphérique. Le système de compression 6 élève la pression de l'effluent réactionnel à une valeur telle qu'une séparation efficace d'une phase hydrocarbonée liquide d'une phase gazeuse riche en hydrogène devient possible à une température généralement inférieure à 0°C. Dans le cas de la déshydrogénation de l'isobutane, une pression de 13 à 18 bar est tout à fait appropriée. Les besoins en énergie du système de compression peuvent être fournis par une turbine à gaz, une turbine à vapeur ou un moteur électrique.

A la sortie du compresseur 6, une ligne 7 divise l'effluent comprimé et saturé en eau en deux flux. Un premier flux représentant 5 à 10 % de l'effluent est dirigé par une ligne 11 vers la partie inférieure d'une colonne 10 d'inhibition de l'eau (ou de strippage) contenant un garnissage structuré par exemple un garnissage SULZER BX ayant 5 à 10 plateaux théoriques équivalents.

Ce flux est mis en contact ascendant à contre-courant avec une alimentation 9 en eau et en méthanol en phase liquide introduite en tête de colonne et provenant d'une enceinte de séparation 8 définie ci-après.

A la sortie inférieure, on récupère une phase aqueuse ne contenant sensiblement plus de solvant, que l'on recycle à l'entrée supérieure de la colonne de saturation 3. A la sortie supérieure de la colonne de strippage, une ligne 47 évacue une phase gazeuse chargée en solvant et en effluent hydrocarboné vers la partie restante de l'effluent comprimé, saturé en eau et refroidi par un échangeur thermique 51 à air ou à eau et le mélange effluent-méthanol-eau est refroidi par un échange indirect de chaleur dans un échangeur 19 avec un effluent 18 d'une enceinte de séparation 8 contenant les hydrocarbures oléfiniques (plus de 50% par exemple) et saturés non convertis. Puis, ce mélange dont l'eau présente est inhibée par la présence de méthanol en quantité adéquate est introduit dans un échangeur de chaleur 13 à tubes et à calandre adapté à réfrigérer l'effluent par échange indirect. En effet la charge d'isobutane liquide fraîche et non convertie donc recyclée est amenée par une ligne 12 en phase liquide et refroidie dans un échangeur thermique 28 à la base de la calandre de l'échangeur thermique 13. La présence d'eau dans la charge peut être inhibée par ajout de méthanol 12a dans la ligne 12. Cette phase liquide est vaporisée par la chaleur cédée par les tubes en présence d'une partie au moins d'hydrogène recyclé introduit par une ligne 15 dans la calandre, qui contribue à abaisser sa température d'ébullition à une pression donnée. L'hydrogène est introduit à la partie inférieure de la calandre par des moyens 61 adaptés à favoriser le contact avec ladite phase liquide sensiblement dans tout le volume occupé par celle-ci. Elle sert dans ces conditions de fluide réfrigérant. Une phase gazeuse est récupérée dans la partie supérieure de la calandre, comprenant l'isobutane vaporisé et l'hydrogène recyclé et évacuée par une ligne 2 en direction de l'échangeur de chaleur à plaques 41 en amont du réacteur 40 de façon à refroidir l'effluent. On peut récupérer dans la partie inférieure 13a de la calandre un condensat de méthanol et d'eau qui peut être recyclé par une ligne 50 à l'alimentation 9 de la colonne de strippage 10.

La pression à l'intérieur de la calandre de l'échangeur de chaleur 13 adapté à réfrigérer l'effluent et donc la pression de vaporisation est maintenue à un niveau approprié par un compresseur 14 à simple étage et à vitesse variable qui a son entrée connectée directement à la calandre.

Pour obtenir une gamme avantageuse de températures de -25°C à -10°C, la pression dans la calandre est maintenue à environ 2 bar absolus. Par l'intermédiaire de ce compresseur 14, la charge en phase vapeur (isobutane et hydrogène) est comprimée vers l'entrée du réacteur de déshydrogénation à une pression de 3 à 4 bar absolus.

L'effluent réfrigéré dans les tubes de l'échangeur de chaleur 13 sort par une ligne 16 à une température d'environ -20 à -25°C par exemple et est dirigé vers un séparateur 8 de phases.

En tête du séparateur, une ligne 22 récupère une phase vapeur contenant de l'hydrogène en majeure partie et une faible proportion d'hydrocarbures et la conduit dans un turbodétendeur 23 où elle est détendue à entropie constante d'une pression par exemple de 15 bar à une pression d'environ 2,5 bar. A la sortie du turbodétendeur 23, la température dans la ligne 26 chute typiquement, de -25°C environ à -85°C, ce qui provoque la condensation d'une phase liquide hydrocarbonée dite cryogénique qui est séparée de l'hydrogène dans une enceinte de séparation 27. Cette phase liquide est récupérée en fond de l'enceinte 27 par une ligne 17 et mélangée à l'effluent réfrigéré sortant de l'échangeur de chaleur 13 réfrigérant, en un point en amont de l'entrée du séparateur 8. Ce contact direct par des moyens de mélange appropriés contribue à refroidir l'effluent comprimé d'environ 2 à 10°C supplémentaires.

A la partie supérieure de l'enceinte de séparation 27, on récupère une phase gazeuse d'hydrogène (plus de 98 % en moles d'hydrogène) qui est divisée pour former un courant d'hydrogène destiné à être recyclé en partie par une ligne 15 et envoyé tout d'abord dans la calandre de l'échangeur de chaleur 13 réfrigérant pour vaporiser la charge liquide d'isobutane. L'autre partie du courant d'hydrogène est dirigée par une ligne 25 vers un compresseur 24 mis en mouvement par le turbodétendeur 23. Ce courant d'hydrogène comprimé à 10 bar environ et à une température de -30°C peut être réchauffé dans un échangeur de chaleur 28 disposé sur la ligne 12 en aval de l'ajout 12a de méthanol, par échange indirect avec la charge liquide d'isobutane, et peut contribuer de ce fait au refroidissement de la charge avant sa vaporisation.

En fond du séparateur, on récupère par une ligne 18 une phase liquide hydrocarbonée à environ -25°C et 15 bar absolus environ, comprenant au moins 95 % d'isobutène contenu dans l'effluent, qui refroidit dans un échangeur de chaleur 19 l'effluent hydrocarboné avant son entrée dans l'échangeur de chaleur réfrigérant 13. La phase liquide hydrocarbonée est à nouveau réchauffée dans un autre échangeur de chaleur 21 avant d'être introduite dans une colonne de stabilisation 20 délivrant en tête du fuel gaz par une ligne 33 et en fond par une ligne 34 de l'isobutène et de l'isobutane non converti qui contribuent à l'échange thermique dans l'échangeur 21 et que l'on envoie dans une unité de formation de MTBE (non représentée).

Par ailleurs, on récupère dans une enceinte 30 en fond du séparateur 8 une phase liquide décantée d'eau contenant 20 à 70 % en poids de méthanol que l'on recycle par une ligne 9 et une pompe 49 vers l'entrée supérieure de la colonne de strippage.

La concentration en méthanol à introduire dans l'effluent hydrocarboné devant être réfrigéré peut être contrôlée en permanence par un densimètre 31 plongé dans l'enceinte de décantation 30 du séparateur 8 qui permet des mesures de densité puis de concentration. Le densimètre est relié à un micro-ordinateur 32 qui commande par comparaison avec une gamme de concentrations préalablement enregistrée une vanne d'ouverture ou de fermeture 60 d'une conduite d'alimentation 36 en un fluide contenant du méthanol, vers la ligne 47.

Selon la figure 2 qui illustre une variante de l'unité de déshydrogénation catalytique d'une coupe isobutane, le dispositif de saturation et d'inhibition de l'eau par le méthanol est identique à celui représenté sur la figure 1 avec les mêmes références. On a cependant décrit le cas où la charge d'isobutane liquide est introduite par la ligne 12 dans un échangeur thermique 60 où elle est vaporisée en l'absence d'hydrogène par échange indirect avec l'effluent hydrocarboné provenant du réacteur de déshydrogénation et contenant la proportion adéquate de méthanol selon l'invention.

L'effluent est encore refroidi dans l'échangeur thermique 19 par échange indirect avec les oléfines et les hydrocarbures non convertis provenant du séparateur 8 par la ligne 18 puis est réfrigéré entre -20 et -40°C dans un échangeur thermique 13 indirect par un dispositif 35 de réfrigération externe, par exemple au propane, avant d'être acheminé dans le séparateur 8 par la ligne 16. L'hydrogène est récupéré en tête par la ligne 22 tandis que les oléfines et du fuel gaz sont récupérés par la ligne 18 pour être séparés dans la colonne de stabilisation 20.

La phase décantée dans l'enceinte inférieure 30 du séparateur 8 contenant la phase liquide eau et méthanol est recyclée comme décrit ci-avant par la ligne 9 vers la colonne de strippage 10.

## Revendications

1. Procédé de déshydrogénation catalytique d'une charge hydrocarbonée paraffinique C₂+ comprenant une étape de déshydrogénation de ladite charge en phase gazeuse éventuellement en présence d'hydrogène, délivrant un effluent gazeux de déshydrogénation comprenant de l'eau, de l'hydrogène, des hydrocarbures oléfiniques et des hydrocarbures paraffiniques non convertis, au moins une étape de refroidissement de l'effluent, une étape de compression de l'effluent refroidi à une pression appropriée, une étape d'inhibition de l'eau contenue dans l'effluent comprimé, une étape de réfrigération dans un échangeur de chaleur de l'effluent comprimé, une étape de séparation de l'effluent comprimé et réfrigéré dans une zone de séparation dans des conditions adéquates et une étape de récupération d'hydrogène et d'hydrocarbures en majeure partie oléfiniques, le procédé étant caractérisé en ce qu'on sature en eau une partie au moins de l'effluent refroidi dans des conditions adéquates dans une zone de saturation par contact direct avec un mélange de phases liquides contenant en majeure partie de l'eau, avant ou après l'étape de compression et on effectue l'étape d'inhibition de l'eau dans l'effluent :
a) en contactant dans une zone de strippage et dans des conditions adéquates une partie au moins de l'effluent comprimé et saturé en eau avec une phase liquide recyclée renfermant à la fois de l'eau et un solvant dans des proportions appropriées, ledit solvant étant un composé organique nonhydrocarbure, normalement liquide, autre que l'eau, ledit composé étant au moins partiellement miscible à l'eau et distillable à une température inférieure à celle de la distillation de l'eau, de façon à obtenir une phase gazeuse chargée de solvant et d'effluent et une phase liquide aqueuse sensiblement débarrassée de solvant ;
b) en introduisant la phase gazeuse chargée de solvant et d'effluent dans la partie restante de l'effluent comprimé et saturé en eau, lorsque la totalité de l'effluent comprimé et saturé en eau n'a pas été mis en contact avec la phase liquide recyclée ;
c) en faisant circuler la phase gazeuse chargée de solvant et d'effluent de l'étape b) ou de l'étape a) lorsque l'étape b) n'est pas nécessaire, dans ledit échangeur de chaleur lors de l'étape de réfrigération ;
d) en séparant dans la zone de séparation une phase liquide décantée contenant de l'eau et du solvant avec une concentration appropriée et
e) en récupérant et en recyclant la phase liquide décantée de l'étape d) vers ladite colonne de strippage de l'étape a).

2. Procédé selon la revendication 1 dans lequel le solvant est choisi dans le groupe formé par le méthylpropyléther, l'éthylpropyléther, le dipropyléther, le méthyltertiobutyléther, le diméthoxyméthane, le diméthoxyéthane, l'éthanol, le méthoxyéthanol, le propanol et le méthanol.

3. Procédé selon la revendication 1 dans lequel le solvant est le méthanol.

4. Procédé selon l'une des revendications 1 à 3 dans lequel on introduit 1 à 100 % en poids de l'effluent comprimé et saturé en eau dans la colonne de strippage et de préférence 10 à 50 %.

5. Procédé selon l'une des revendications 1 à 4 dans lequel la colonne de strippage comprend un garnissage de préférence structuré de hauteur équivalente à au mois 1 plateau théorique, de préférence 10 à 15 plateaux, de façon que la phase liquide aqueuse renferme au plus 10 000 ppm de solvant et de préférence moins de 2 000 ppm.

6. Procédé selon l'une des revendications 1 à 5 dans lequel la concentration de solvant dans la phase liquide décantée est de 20 à 80 % en poids.

7. Procédé selon l'une des revendications 1 à 6 dans lequel on ajuste la concentration de solvant dans l'effluent comprimé par l'ajout d'un flux contenant du solvant de telle façon que la concentration de solvant dans la phase décantée soit de 20 à 80 % en poids.

8. Procédé selon l'une des revendications 1 à 7 dans lequel on effectue la saturation de l'effluent refroidi en introduisant à la partie supérieure de la zone de saturation la phase liquide aqueuse récupérée de la colonne de strippage et une phase aqueuse recyclée récupérée en fond de la zone de saturation, en introduisant à la partie inférieure de la zone de saturation l'effluent refroidi, en réalisant un contact direct à contre-courant de l'effluent avec le mélange des phases aqueuses à travers un garnissage approprié, en récupérant la phase aqueuse en fond de la zone de saturation que l'on recycle, après l'avoir refroidie au moins une fois à la partie supérieure de ladite zone, et on récupère dans la partie supérieure de la zone de saturation l'effluent saturé en eau et refroidi.

9. Procédé selon la revendication 8, dans lequel la température dans la zone de saturation est de 0 à 100°C et la pression est de 1 à 35 bar absolus.

10. Procédé selon l'une des revendications 1 à 9 dans lequel le mélange des phases aqueuses renferme en outre de 1 à 15 % d'un hydroxyde de métal alcalin et de préférence de la soude.

11. Procédé selon l'une des revendications 1 à 10 dans lequel la température dans la zone de strippage est de 10 à 150°C sous une pression de 3 à 35 bar absolus et de préférence 80 à 120°C, sous une pression de 10 à 15 bar.

12. Procédé selon l'une des revendications 1 à 11 dans lequel la zone de séparation délivrant la phase liquide décantée qui est régulée est à une température de 0 à -100°C sous 3 à 35 bar absolus et avantageusement - 20 à - 40°C sous 8 à 12 bar absolus.

13. Unité de déshydrogénation catalytique comprenant en combinaison un réacteur (40) de déshydrogénation délivrant un effluent contenant de l'eau, des moyens d'alimentation (12, 2) en une charge hydrocarbonée connectés à une entrée du réacteur, au moins un moyen de refroidissement (41) d'un effluent relié à une sortie du réacteur, des moyens de compression (6) de l'effluent, des moyens d'inhibition de l'eau contenue dans l'effluent, des moyens de réfrigération (13) de l'effluent comprimé reliés aux moyens d'inhibition de l'eau, des moyens de séparation (8) de l'effluent réfrigéré relié aux moyens de réfrigération, des moyens de récupération (22, 18) d'une phase riche en hydrogène et d'une phase liquide contenant des hydrocarbures oléfiniques, ladite unité étant caractérisée en ce qu'elle comporte :
- des moyens de saturation en eau de l'effluent comprenant une enceinte allongée ayant un garnissage approprié (3) qui comporte à une extrémité une première entrée reliée à une alimentation (1) en effluent, une première sortie d'eau reliée à des moyens de recyclage (42, 45) de l'eau, à l'autre extrémité une deuxième entrée reliée aux moyens de recyclage (42) de l'eau et une deuxième sortie (5) délivrant l'effluent saturé en eau, et
- les moyens d'inhibition de l'eau comportant une colonne (10) de strippage ayant un garnissage adéquat, de préférence structuré, et ayant à une première extrémité une première entrée (11) de l'effluent saturé en eau reliée à la deuxième sortie de l'enceinte (3) de saturation en eau de l'effluent, une première sortie reliée aux moyens (42a) de recyclage de l'eau vers l'enceinte (3) de saturation et à une extrémité opposée une deuxième entrée reliée à des moyens de recyclage (9) d'une phase liquide aqueuse contenant du solvant connectés auxdits moyens de séparation (8) adaptés à délivrer ladite phase liquide aqueuse et une deuxième sortie (47) connectée aux moyens de réfrigération (13) de l'effluent.

14. Unité de déshydrogénation selon la revendication 13 dans laquelle la première entrée de l'enceinte (3) de saturation est reliée auxdits moyens (41) de refroidissement de l'effluent, la deuxième sortie de l'enceinte de saturation est reliée à une entrée des moyens de compression (6) et la première entrée de l'effluent saturé en eau dans la colonne (10) de strippage est connectée à une sortie (7) des moyens de compression.

15. Unité de déshydrogénation selon la revendication 13 dans laquelle les moyens (41) de refroidissement de l'effluent sont connectés à une entrée des moyens (6) de compression, la première entrée de l'enceinte (3) de saturation est reliée à une sortie des moyens de compression (6), et la deuxième sortie de l'enceinte de saturation (3) est reliée directement à la première entrée de la colonne (10) de strippage.

16. Unité de déshydrogénation selon la revendication 15 dans laquelle la deuxième sortie de l'enceinte (3) de saturation est reliée en outre aux moyens de réfrigération.

17. Unité de déshydrogénation selon la revendication 14 dans laquelle la sortie des moyens de compression (6) est connectée en outre aux moyens de réfrigération (13).

18. Unité de déshydrogénation selon l'une des revendications 13 à 17 dans laquelle les moyens de réfrigération comprennent un échangeur (13) indirect de chaleur à double enceinte, la première enceinte comportant une alimentation (12) en charge liquide, éventuellement une alimentation (15) en hydrogène recyclé gazeux, ladite première enceinte étant adaptée à vaporiser la charge et à réfrigérer ladite seconde enceinte où circule l'effluent par échange indirect et comportant en outre une sortie reliée aux moyens (2) d'alimentation du réacteur (40).

19. Unité de déshydrogénation selon l'une des revendications 13 à 17 dans laquelle les moyens de réfrigération de l'effluent comprennent un échangeur de chaleur (13, 35) utilisant un fluide de réfrigération externe.

20. Unité de déshydrogénation selon l'une des revendications 13 à 19 dans laquelle les moyens (42, 45) de recyclage en eau vers l'enceinte de saturation comportent au moins un moyen (43) de refroidissement de l'eau.

## Patentansprüche

1. Verfahren zur katalytischen Dehydrierung einer kohlenwasserstoffhaltigen paraffinischen C₂₊-Charge, eine Stufe der Dehydrierung dieser Charge in gasförmiger Phase, gegebenenfalls in Anwesenheit von Wasserstoff umfassend, unter Lieferung eines gasförmigen Dehydrierungsabstroms, der Wasser, Wasserstoff, olefinische Kohlenwasserstoffe und nicht umgesetzte paraffinische Kohlenwasserstoffe umfaßt, wenigstens eine Stufe der Kühlung dieses Abstroms, eine Stufe der Kompression dieses gekühlten Abstroms bei einem geeigneten Druck, eine Stufe der Inhibierung von im komprimierten Abstrom enthaltenem Wasser, eine Stufe der Kühlung in einem Wärmeaustauscher für den komprimierten Abstrom, eine Stufe zur Trennung des komprimierten und gekühlten Abstroms in einer Trennzone unter adäquaten Bedingungen und eine Stufe der Rückgewinnung von Wasserstoff und, zum größeren Teil olefinischen, Kohlenwasserstoffen, umfassend, wobei das Verfahren sich dadurch auszeichnet, daß man mit Wasser wenigstens einen Teil des gekühlten Abstroms unter adäquaten Bedingungen in einer Sättigungszone durch direkten Kontakt mit einem Gemisch aus flüssigen Phasen, die zum größeren Teil Wasser enthalten, vor oder nach der Stufe der Kompression sättigt und man die Stufe der Inhibierung des Wassers im Abstrom durchführt:
a) indem man in einer Stripperzone und unter adäquaten Bedingungen wenigstens einen Teil des komprimierten und mit Wasser gesättigten Abstroms mit einer flüssigen rezyklierten Phase kontaktiert, die gleichzeitig Wasser und ein Lösungsmittel in geeigneten Anteilen einschließt, wobei dieses Lösungsmittel eine organische nicht kohlenwasserstoffhaltige Zusammensetzung, die normalerweise flüssig ist, außer Wasser, ist, und die Zusammensetzung wenigstens teilweise mit Wasser mischbar und bei einer Temperatur unterhalb der Destillationstemperatur des Wassers destillierbar ist, derart, daß man eine gasförmige mit Lösungsmittel und Abstrom beladene Phase und eine flüssige wäßrige, im wesentlichen von Lösungsmittel befreite Phase erhält;
b) indem man die gasförmige mit Lösungsmittel und Abstrom im verbleibenden Teil des komprimierten und mit Wasser gesättigten Abstroms einführt, wenn die Gesamtheit des komprimierten und mit Wasser gesättigten Abstroms mit der flüssigen rezyklierten Phase nicht kontaktiert wurde;
c) indem man die gasförmige mit Lösungsmittel und Abstrom aus der Stufe b) oder Stufe a), wenn die Stufe b) nicht notwendig ist, beladene Phase in diesem Wärmeaustauscher beim Kühlschritt zirkulieren läßt;
d) indem man in der Trennzone eine flüssige dekantierte Wasser und Lösungsmittel mit einer geeigneten Konzentration enthaltende Phase trennt und
e) indem man die flüssige dekantierte Phase aus der Stufe d) rückgewinnt und zu dieser Stripperkolonne der Stufe a) rezykliert.

2. Verfahren nach Anspruch 1, bei dem das Lösungsmittel gewählt ist aus der Gruppe, die durch Methylpropylether, Ethylproplyether, Dipropylether, Methyl-tert.-buthylether, Dimethyloxymethan, Dimethyloxyethan, Ethanol, Methoxyethanol, Propanol und Methanol gebildete ist.

3. Verfahren nach Anspruch 1, bei dem das Lösungsmittel Methanol ist.

4. Verfahren nach einem der Ansprüche 1 bis 3, bei dem man 1 bis 100 Gew.-% des komprimierten Abstroms, der mit Wasser gesättigt ist, in die Stripperkolonne, bevorzugt 10 bis 50 %, einführt.

5. Verfahren nach einem der Ansprüche 1 bis 4, bei dem die Stripperkolonne eine Auskleidung umfaßt, die bevorzugt mit der äquivalenten Höhe auf wenigstens einen theoretischen Boden, bevorzugt 10 bis 15 Böden, strukturiert ist, derart, daß die flüssige wäßrige Phase höchstens 10 000 ppm Lösungsmittel und bevorzugt wenigstens 2 000 ppm umschließt.

6. Verfahren nach einem der Ansprüche 1 bis 5, bei dem die Lösungsmittelkonzentration in der flüssigen dekantierten Phase 20 bis 80 Gew.-% beträgt.

7. Verfahren nach einem der Ansprüche 1 bis 6, bei dem man die Lösungsmittelkonzentration im komprimierten Abstrom durch Zugabe eines Flußmittels einstellt, das Lösungsmittel derart enthält, daß die Lösungsmittelkonzentration in der dekantierten Phase 20 bis 80 Gew.-% beträgt.

8. Verfahren nach einem der Ansprüche 1 bis 7, bei dem man die Sättigung des gekühlten Lösungsmittels vornimmt, indem man am oberen Teil der Sättigungszone die flüssige wäßrige Phase, die aus der Stripperkolonne rückgewonnen wurde, sowie eine wäßrige rezyklierte am Boden der Sättigungszone rückgeführte Phase einführt, indem man am unteren Teil der Sättigungszone den gekühlten Abstrom einführt, indem man einen direkten Gegenstromkontakt des Abstroms mit dem Gemisch der wäßrigen Phasen durch eine geeignete Auskleidung realisiert, indem man die wäßrige Phase am Boden der Sättigungszone, die man rezykliert, gewinnt, nachdem man sie wenigstens einmal im oberen Teil dieser Zone gekühlt hat und man im oberen Teil der Sättigungszone den mit Wasser gesättigten und gekühlten Abstrom gewinnt.

9. Verfahren nach Anspruch 8, bei dem die Temperatur in der Sättigungszone 0 bis 100°C und der Druck 1 bis 35 bar absolut beträgt.

10. Verfahren nach einem der Ansprüche 1 bis 9, bei dem das Gemisch der wäßrigen Phasen im übrigen 1 bis 15 % eines Alkalimetallhydroxyds und bevorzugt Natriumkarbonat einschließt.

11. Verfahren nach einem der Ansprüche 1 bis 10, bei dem die Temperatur in der Stripperzone 10 bis 150°C unter einem Druck von 3 bis 35 bar absolut und bevorzugt 80 bis 120°C unter einem Druck von 10 bis 15 bar beträgt.

12. Verfahren nach einem der Ansprüche 1 bis 11, bei dem die Trennzone die flüssige dekantierte Phase liefert, die auf eine Temperatur von 0 bis -100°C unter 3 bis 35 bar absolut und bevorzugt -20 bis -40°C unter 8 bis 12 bar absolut eingestellt worden ist.

13. Katalytische Dehydrierungseinheit, in Kombination einen Dehydrierungsreaktor (40) umfassend, der einen Wasser enthaltenden Abstrom liefert, Mittel zur Speisung (12, 2) mit einer kohlenwasserstoffhaltigen Charge, die mit einem Eingang des Reaktors verbunden sind, wenigstens ein Kühlmittel (41) für einen Abstrom, das mit einem Ausgang des Reaktors verbunden ist, Mittel zur Kompression (6) dieses Abstroms, Mittel zur Inhibierung des im Abstrom enthaltenen Wassers, Kühlmittel (13) für den komprimierten Abstrom, die mit den Wasserinhibierungsmitteln verbunden sind, Mittel (8) zur Trennung des gekühlten Abstroms, in Verbindung mit den Kühlmitteln, Rückgewinnungsmittel (22, 18) für eine an Wasserstoff reiche und eine flüssige Phase, die olefinische Kohlenwasserstoffe enthält, wobei diese Einheit sich dadurch auszeichnet, daß sie umfaßt:
- Mittel zur Sättigung des Abstroms mit Wasser, eine längliche Kammer mit einer geeigneten Auskleidung (3) umfassend, die an einem Ende einen ersten mit einer Speisung (1) mit Abstrom verbundenen Eingang, einen ersten mit Rezyklierungsmitteln (42, 45) für das Wasser verbundenen Wasserausgang umfaßt, am anderen Ende einen zweiten Eingang, der mit den Rezyklierungsmitteln (42) für das Wasser verbunden ist und einen zweiten Ausgang (5), der den mit Wasser gesättigten Abstrom liefert, umfaßt, und
- die Wasserinhibierungsmittel eine Stripperkolonne (10) umfassen, die eine adäquate Auskleidung, bevorzugt strukturiert, hat und an einem ersten Ende einen ersten Eingang (11) für den mit Wasser gesättigten Abstrom hat, der mit dem zweiten Ausgang der mit Wasser gesättigten Kammer (3) des Abstroms verbunden ist, einen ersten mit den Rezyklierungsmitteln des Wassers zur Sättigungskammer (3) verbundenen Ausgang und an einem gegenüberliegenden Ende einem zweiten Eingang hat, der mit Rezyklierungsmitteln (9) einer flüssigen wäßrigen Phase verbunden ist, die Lösungsmittel enthält, wobei diese mit den Trennmitteln (8) verbunden sind, die so ausgelegt sind, daß sie diese flüssige wäßrige Phase liefern und ein zweiter Ausgang (47) mit den Kühlmitteln (13) für den Abstrom verbunden ist.

14. Dyhdrierungseinheit nach Anspruch 13, bei der der erste Eingang der Sättigungskammer (3) mit diesen Kühlmitteln (41) für den Abstrom verbunden ist, der zweite Ausgang der Sättigungskammer mit einem Eingang der Kompressionsmittel (6) verbunden ist und der erste Eingang für den mit Wasser gesättigten Abstrom in die Stripperkolonne (10) mit einem Ausgang (7) der Kompressionsmittel verbunden ist.

15. Dehydrierungseinheit nach Anspruch 13, bei der die Kühlmittel (41) des Abstroms mit einem Eingang der Kompressionsmittel (6) verbunden sind, der erste Eingang der Sättigungskammer (3) mit einem Ausgang der Kompressionsmittel (6) verbunden ist und der zweite Ausgang der Sättigungskammer (3) direkt mit dem ersten Eingang der Stripperkolonne (10) verbunden ist.

16. Dehydrierungseinheit nach Anspruch 15, bei der der zweite Ausgang der Sättigungskammer (3) im übrigen mit den Kühlmitteln verbunden ist.

17. Dehydrierungseinheit nach Anspruch 14, bei der der Ausgang der Kompressionsmittel (6) im übrigen mit den Kühlmitteln (13) verbunden ist.

18. Dehydrierungseinheit nach einem der Ansprüche 13 bis 17, bei der die Kühlmittel einen indirekten Wärmeaustauscher (13) mit Doppelkammer umfassen, die erste Kammer eine Speisung (12) mit flüssiger Charge, gegebenenfalls eine Speisung (15) mit gasförmigem rezykliertem Wasserstoff umfaßt, wobei die erste Kammer so ausgelegt ist, daß sie die Charge verdampft und diese zweite Kammer kühlt, wo der Abstrom mit indirektem Austausch zirkuliert und im übrigen einen Ausgang umfaßt, der mit den Speisemitteln (2) des Reaktors (40) verbunden ist.

19. Dehydrierungseinheit nach einem der Ansprüche 13 bis 17, bei der die Kühlmittel für den Abstrom einen Wärmeaustauscher (13, 35) umfassen, der ein äußeres Kühlfluid nutzt.

20. Dehydrierungseinheit nach einem der Ansprüche 13 bis 19, in der die Mittel (42, 45) zur Wasserrezyklierung zur Sättigungskammer wenigstens ein Wasserkühlmittel (43) umfassen.

## Claims

1. Process of catalytic dehydrogenation of a C₂₊ paraffinic hydrocarbon charge comprising a step of dehydrogenating said charge in gas phase optionally in the presence of hydrogen, delivering a dehydrogenation gaseous effluent comprising water, hydrogen, olefinic hydrocarbons and unconverted paraffinic hydrocarbons, at least one step of cooling the effluent, a step of compressing the cooled effluent at a suitable pressure, a step of inhibiting the water contained in the compressed effluent, a step of cooling the compressed effluent in a heat exchanger, a step of separating the compressed and cooled effluent in a separation zone under suitable conditions and a step of recovering hydrogen and hydrocarbons, for the most part olefinic, the process being characterized in that at least one part of the cooled effluent is saturated with water under suitable conditions in a saturation zone by direct contact with a mixture of liquid phases containing mostly water, before or after the compression step, and the step of inhibiting the water in the effluent is performed:
a) by contacting in a stripping zone and under suitable conditions at least one part of the effluent that is compressed and saturated with water with a recycled liquid phase containing both water and a solvent in suitable proportions, said solvent being a nonhydrocarbon organic compound, normally liquid, other than water, said compound being at least partially water-miscible and distillable at a temperature lower than that of the distillation of water, to obtain a gas phase charged with solvent and effluent and an aqueous liquid phase essentially rid of solvent;
b) by introducing the gas phase that is charged with solvent and effluent into the remaining part of the effluent that is compressed and saturated with water, when the whole of the effluent that is compressed and saturated with water has not been put in contact with the recycled liquid phase;
c) by making the gas phase that is charged with solvent and effluent of step b), or of step a) when step b) is not necessary, circulate in said heat exchanger during the cooling step;
d) by separating in the separation zone a decanted liquid phase containing water and solvent with a suitable concentration and
e) by recovering and by recycling the decanted liquid phase of step d) to said stripping column of step a).

2. Process according to claim 1, wherein the solvent is selected from the group formed by methyl propyl ether, ethyl propyl ether, dipropyl ether, methyl tert-butyl ether, dimethoxymethane, dimethoxyethane, ethanol, methoxyethanol, propanol and methanol.

3. Process according to claim 1, wherein the solvent is methanol.

4. Process according to one of claims 1 to 3, wherein 1 to 100% by weight of the effluent that is compressed and saturated with water is introduced into the stripping column and preferably 10 to 50%.

5. Process according to one of claims 1 to 4, wherein the stripping column comprises a preferably structured packing of a height equivalent to at least 1 theoretical plate, preferably 10 to 15 plates, so that the aqueous liquid phase contains at most 10,000 ppm of solvent and preferably less than 2,000 ppm.

6. Process according to one of claims 1 to 5, wherein the concentration of solvent in the decanted liquid phase is 20 to 80% by weight.

7. Process according to one of claims 1 to 6, wherein the concentration of solvent in the compressed effluent is adjusted by adding a flow containing solvent so that the concentration of solvent in the decanted phase is 20 to 80% by weight.

8. Process according to one of claims 1 to 7, wherein the saturation of the cooled effluent is performed by introducing the aqueous liquid phase recovered from the stripping column and a recycled aqueous phase recovered at the bottom of the saturation zone at the upper part of the saturation zone, by introducing the cooled effluent at the lower part of the saturation zone, by producing a direct countercurrent contact of the effluent with the mixture of aqueous phases through a suitable packing, by recovering the aqueous phase at the bottom of the saturation zone that is recycled, after having cooled it at least once, at the upper part of said zone, and the water-saturated and cooled effluent is recovered in the upper part of the saturation zone.

9. Process according to claim 8, wherein the temperature in the saturation zone is 0 to 100°C and the pressure is 1 to 35 absolute bars.

10. Process according to one of claims 1 to 9, wherein the mixture of aqueous phases further contains 1 to 15% of an alkaline metal hydroxide and preferably soda.

11. Process according to one of claims 1 to 10, wherein the temperature in the stripping zone is 10 to 150°C under a pressure of 3 to 35 absolute bars and preferably 80 to 120°C, under a pressure of 10 to 15 bars.

12. Process according to one of claims 1 to 11, wherein the separation zone delivering the decanted liquid phase which is regulated is at a temperature of 0 to -100°C under 3 to 35 absolute bars and advantageously -20 to -40°C under 8 to 12 absolute bars.

13. Catalytic dehydrogenation unit comprising in combination a dehydrogenation reactor (40) delivering an effluent containing water, means (12, 2) for feeding a hydrocarbon charge connected to an input of the reactor, at least one means (41) for cooling an effluent connected to an output of the reactor, means (6) for compressing the effluent, means for inhibiting the water contained in the effluent, means (13) for cooling the compressed effluent connected to the means for inhibiting the water, means (8) for separating the cooled effluent connected to the cooling means, means (22, 18) for recovering a hydrogen-rich phase and a liquid phase containing olefinic hydrocarbons, said unit being characterized in that it comprises:
-- means for water saturation of the effluent comprising an elongated chamber having a suitable packing (3), which comprises at one end a first input connected to an effluent feed (1), a first water output connected to means (42, 45) for recycling the water, at the other end a second input connected to means (42) for recycling the water and a second output (5) delivering the water-saturated effluent, and
-- means for inhibiting the water comprising a stripping column (10) having a suitable packing, preferably structured, and having at a first end a first input (11) of the water-saturated effluent connected to the second output of chamber (3) for water saturation of the effluent, a first output connected to means (42a) for recycling the water to saturation chamber (3) and at an opposite end, a second input connected to means (9) for recycling an aqueous liquid phase containing solvent connected to said separating means (8) suited to deliver said aqueous liquid phase and a second output (47) connected to means (13) for cooling the effluent.

14. Dehydrogenation unit according to claim 13, wherein the first input of saturation chamber (3) is connected to said means (41) for cooling the effluent, the second output of the saturation chamber is connected to an input of compression means (6) and the first input of the water-saturated effluent in stripping column (10) is connected to an output (7) of the compression means.

15. Dehydrogenation unit according to claim 13, wherein means (41) for cooling the effluent are connected to an input of compression means (6), the first input of saturation chamber (3) is connected to an output of compression means (6), and the second output of saturation chamber (3) is connected directly to the first input of stripping column (10).

16. Dehydrogenation unit according to claim 15, wherein the second output of saturation chamber (3) is further connected to the cooling means.

17. Dehydrogenation unit according to claim 14, wherein the output of compression means (6) is further connected to cooling means (13).

18. Dehydrogenation unit according to one of claims 13 to 17, wherein the cooling means comprise a dual-chamber indirect heat exchanger, the first chamber comprising a feed (12) of liquid charge, optionally a feed (15) of gaseous recycled hydrogen, said first chamber being suited to evaporate the charge and to cool said second chamber where the effluent circulates by indirect exchange and further comprising an output connected to means (2) for feeding reactor (40).

19. Dehydrogenation unit according to one of claims 13 to 17, wherein the means for cooling the effluent comprise a heat exchanger (13, 35) using an external cooling fluid.

20. Dehydrogenation unit according to cne of claims 13 to 19, wherein means (42, 45) for recycling water to the saturation chamber comprise at least one means (43) for cooling the water.
